# EUROPEAN PATENT SPECIFICATION

(11) **EP 0 374 823 B1**
(45) Date of publication and mention of the grant of the patent: **08.12.1993**
(21) Application number: 89123445.2
(22) Date of filing: 19.12.1989
(51) Int. Cl.: A01N 63/02, A23L 3/3571

(54) **Control of microbial growth with nisin/lysozyme formulations**
Kontrolle des Mikrobenwachstums mit Nisin/Lysozym-Formulierungen
Contrôle de la croissance microbienne avec des formulations de nisine et lysozyme

(30) Priority: 21.12.1988 US 287719; 16.11.1989 US 434671
(43) Date of publication of application: 27.06.1990
(73) Proprietor: HAARMANN & REIMER CORP., Springfield, New Jersey 07081 (US)
(72) Inventor: Monticello, Daniel J., Elkhart Indiana 46514 (US)
(74) Representative: Petrovicki, Wolfgang, Dr.

(56) References cited:
- EP-A- 0 265 884
- A.L. BRANEN et al.: "Antimicrobials in Food", 1983, pages 327-351,381-384, Marcel Dekker Inc., New York, US

## Description

This is a Continuation-In-Part of co-pending application Serial No. 287,719, filed December 21, 1988.

### BACKGROUND OF THE INVENTION

Nisin is an antimicrobial peptide produced by certain strains of Lactococcus lactis (formerly Streptococcus lactis). It is manufactured through the pure-culture fermentation of these bacteria with subsequent purification and drying. The first reported use of nisin as a food preservative occurred in 1951 when it was used to control the growth of Clostridium butyricum and C. tyrobutyricum in cheese (Hirsch et al, 1951, J. Dairy Research 18:205-206).

Nisin normally occurs as a dimer with a molecular weight of about 7000. It contains several unusual amino acids including β-methyllanthionine, dehydroalanine, and lanthionine among its total of 34 amino acids. There are five unusual thio-ether linkages in the peptide which contribute to its stability in acid solutions. Nisin is one of the most thoroughly characterized bacteriocins, and shares remarkable homology of structure and action with other bacteriocins, for example Subtilin and epidermin [Buchman et al 1988. J. Bio. Chem. 263 (31):16260-16266]. It is representative of a class of bacteriocins which have been termed lantiotics (Schnell et al. 1988. Nature 333:276-278). Recent reviews of nisin, its physical properties and uses include "Bacteriocins of Lactic Acid Bacteria", T. R. Klaenhammer, 1988. Biochimie 70:337-349, "Nisin", A. Hurst, 1981. Avd. Appl. Microbiol. 27:85-121, and U.S. Patent 4,740,593. Nisin is the collective name describing several closely related substances which exhibit similar amino acid compositions, and some limited range of antibiotic activity. This phenomenon is discussed by E. Lipinska in "Antibiotics and Antibiosis in Agriculture" (M. Woodbine, Ed.) Pp. 103-130.

The use of nisin to combat L. monocytogenes has been reported by M. Doyle; "Effect of Environmental and Processing Conditions on Listeria Monocytogenes", Food Technology, 1988.42(4):169-171. This reference describes the initial inhibition of the organism's growth (for about 12 hours) and reports that L. monocytogenes may grow at a pH level as low as 5.0 and is resistant to alkaline pH with the ability to grow at pH 9.6.

Lysozymes (1,4-β-N acetylhexosaminodase, E.C. 3.2.1.17) from various sources are well characterized enzymes. First discovered in 1922 by W. Fleming, egg white lysozyme was among the first proteins sequenced, the first for which a three dimensional structure was suggested using x-ray crystallography and the first for which a detailed mechanism of action was proposed. Its antimicrobial activity against gram positive bacteria is well documented, for example by V. N. Procter et al in CRC Crit. Reviews in Food Science and Nutrition, 1988, 26(4):359-395. The molecular weight of egg white lysozyme is approximately 14,300 to 14,600, the isoelectric point is pH 10.5-10.7. It is composed of 129 amino acids which are interconnected by four disulfide bridges. Similar enzymes have been isolated and characterized from other sources including such diverse producers as Escherichia coli bacteriophage T4 and human tears. Despite slight differences (for example, the human lysozyme has 130 amino acids) the capacity for hydrolysis of acetylhexosamine polymers remains essentially the same.

Lysozyme is known to kill or inhibit the growth of bacteria and fungi, and is used in Europe to control the growth of the spoilage organism Clostridium tyrobutyricum in cheese. It has also been proposed for use in a variety of other food preservation applications and has been reported to inhibit the growth of (and in some cases kill) Listeria monocytogenes (Hughey et al, 1987, Appl. Environ. Microbiol 53:2165-2170).

Recombinant DNA, protein engineering and chemical modification technologies can be used to effect subtle modifications of peptides and proteins to alter certain aspects of the molecules and to allow expression in new hosts. For example, the expression of nisin in new hosts is described in United States Patent 4,740,593, and the genes for T4, human and egg white lysozyme as well as nisin and other "lantiotics" have been cloned. Human lysozyme has been expressed in Bacillus subtilus and Saccharomyces cerevisiae. These homologous molecules (nisin, epidermin, subtilin, etc. and T4, human and egg white lysozyme) should be considered as synonomous with nisin and lysozyme in the context of the present invention.

While nisin and lysozyme have been shown to individually inhibit the growth of some bacteria and fungi, it is not clear that either lysozyme or nisin by themselves can sufficiently inhibit the growth of these microorganisms when applied in economically feasible concentrations.

### SUMMARY OF THE INVENTION

The present invention is a method of inhibiting the growth of undesirable microorganisms in an environment amenable to their growth which comprises adding to such environment a synergistically effective combination of nisin and lysozyme in an amount sufficient to inhibit such microbial growth.

### DESCRIPTION OF THE INVENTION

The present invention relates to the observation that nisin and lysozyme, in combination, can be used at significantly lower concentrations than either component alone to yield a similar degree of control of certain microorganisms. Nisin and lysozyme have been used separately in a variety of food preservation applications, e.g. they have been used separately to control pathogenic organisms such as Clostridium botulinum and spoilage organisms such as C. tyrobutyricum in dairy products, processed meats, sea foods, fresh and processed vegetables, seafood products, vegetable and fruit juices and beverages (both soft drinks and alcoholic). However, the price of these substances has hindered the commercial acceptance of these applications. The antimicrobial properties of nisin and lysozyme are also useful in non-food applications including the control of certain infections in humans and animals, the control of microbial growth in chemical feedstocks and industrial products and in cleaning applications such as decontamination of surfaces having microbial contamination.

A variety of microorganisms have been reported to be affected by nisin or lysozyme. For nisin these are primarily gram positive bacteria including some species of Clostridia, Bacilli, Lactococci, Staphlococci, Pediococci, Micrococci, Microbacteria, and Lactobacilli genera. Lysozyme has been shown to effect most bacteria either alone or in combination with other agents. A variety of substances have been proposed for use in combination with lysozyme. These include chelating agents such as EDTA and phytic acid, butyl P-hydroxybenzoate, P-hydroxybenzoic esters, amino acids, hydrogen peroxide and organic acids. These are reviewed by Proctor et al, supra. The synergistic combination of lysozyme with benzoic acid or sorbic acid are discussed in German patent application DE 3704-004 filed february 10, 1987, and with gallic acid, phytic acid or betaine and etapoly:lysine in Japanese patent application J6 3109762, filed on October 28, 1986. A bacteriocidal composition containing glucose oxidase, peroxidase, thiocyanate and lysozyme has been proposed in PCT application WO88/102600, published April 21, 1988. Knorr et al report using lysozyme for the lysis of yeast (J. Food Sci., 1979, 44:1362).

The mechanism of action of nisin has not been resolved, but many investigators have linked the lethality of this peptide to irreversible changes in the cell membranes of susceptible organisms. The resistance of most organisms to nisin is thought to be due to the lack of access of the peptide to its site of action, either because of physical exclusion or the lack of appropriate receptor molecules.

The mechanism of cell lysis by n-acetylhexosaminodases such as egg-white lysozyme involves the hydrolytic cleavage of the structural component of the cell wall; in the case of bacteria, the n-acetylmuramic acid-n-acetyl glucosamine polymer, and in the case of fungi, the chitin (a n-acetyl glucosamine polymer). The synergism of nisin and lysozyme may be a result of each component making the other's substrate more accessible. By degrading the structural component of microbial cell walls lysozyme may increase the accessibility of nisin to the sensitive cell membranes which are otherwise buried beneath the cell surface. Based on the mechanisms of action of these antimicrobials and their known target microorganisms, it can be concluded that the growth of many gram positive and gram negative bacteria as well as certain fungi will be inhibited by exposure to the combination of nisin and lysozyme in a similar manner to that which has been demonstrated for L. monocytogenes.

Among the problematical gram positive bacteria, L. monocytogenes is a particular pest because of its ability to grow at refrigerator temperatures and its pathologic nature which can result in serious consequences when ingested. L. monocytogenes has been implicated in several fatalities in recent years, especially following outbreaks of listerosis associated with milk, cole slaw and soft-ripened cheese. The United States Center for Disease Control has estimated that over 1600 cases of listeriosis will occur in the U.S. annually resulting in over 400 deaths. Mothers, unborn children, newly born infants and immune compromised individuals are especially at risk. L. monocytogenes infections can lead to meningitis or septicemia with about 30% of diagnosed cases being fatal.

The present invention is predicated on the discovery that nisin and lysozyme, in combination, inhibit the growth of undesirable microorganisms to a greater degree than either material by itself and that the combined inhibitory effect of these substances is greater than the additive effect observed when using nisin or lysozyme individually. When using nisin as an anti-microbial agent a loading level of from 200 to 300 International Units (IU) per gram or greater of the substrate being treated is usually recommended to achieve maximal effect. Similarly, pure lysozyme is typically employed at a loading of from 20 to 100 micrograms or more per gram of the material being treated. While each of these materials by itself may be effective at these high loadings, such levels are not economically viable for many applications. The discovery that nisin and lysozyme in combination react in a synergistic way to inhibit the growth of undesirable microorganisms facilitates the use of these materials for inhibiting bacterial growth with greater efficacy and economy than either material by itself.

In a typical example, a substrate to be rendered resistant to undesirable microbial growth should preferably contain both nisin and lysozyme. The total amount of nisin will typically range from about 25 IU/ml (or IU/gm in solid systems) of the material being treated up to as much as 2000 IU/ml. In most applications a nisin concentration of from 100 to 500 IU/ml will be sufficient. The ratio of nisin:lysozyme (IU/ml:µg/ml) will range from 9:1 to 1:2 with a ratio of 2:1 being preferred. The concentrations in solid substrates would be the same except they would be converted from a volume to weight basis. Surface treatment can be accomplished by suspending and storing a substrate to be treated in the nisin/lysozyme solution. Alternatively, the substrate may be dipped in the solution or it can be sprayed onto the surface of the substrate. The nisin/lysozyme combination can be incorporated into films or gels which are applied to the substrate's surface or the combination can be incorporated directly into the environment being treated such as by adding it to milk to be used in making cheese. In film coating applications such as dips, films, gels or casings, the initial concentrations of nisin and lysozyme can approach their solubility limits to provide a finished product which after application and diffusion contains residual levels of nisin/lysozyme which are within the proscribed limits. When treating the substrate's surface some routine experimentation may be needed to ascertain the most effective concentrations of nisin/lysozyme.

The lactic acid bacteria, and in particular Lactococcus lactis, are used in the production of fermented food products such as cheese. Since some strains of L. lactis produce nisin, it has been suggested that these organisms be intentionally added in some fermentations so that nisin is distributed throughout the fermented food product. A major concern with this approach is that the nisin concentrations achieved may vary or be too low to be effective. However, by adding lysozyme to the fermenting product, in accordance with the present invention, the amount of nisin needed for effective control is reduced so that the anti-microbial effect of even low concentrations of nisin is realized.

The present invention is further illustrated by the following examples.

### EXAMPLE I

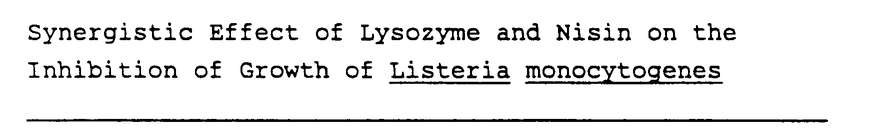

A culture of Listeria monocytogenes Scott A was grown overnight in Brain Heart Infusion broth (BHI, Difco Laboratories, Inc.). Overlay plates were prepared using 25 ml of BHI agar overlayed with 4 ml BHI agar containing 10 µl of the overnight culture. Sterile antibiotic sensitivity disks were blotted with 10 µl of nisin, lysozyme or nisin/lysozyme blends at various concentrations; placed on the surface of the overlay plates and incubated overnight at 37°C.

No inhibition of bacterial growth was observed with nisin at concentrations below 50 IU/ml nor was inhibition observed with lysozyme at concentrations up to and including 125 µg/ml. When a solution with 50 IU/ml nisin and 50 µg/ml lysozyme was used, however, a clear and obvious zone of inhibition was observed around the discs. Since the concentrations of nisin and lysozyme at the outer edge of the zone of inhibition must have been much less than that originally applied to the disk, one can conclude that small quantities of nisin and lysozyme act synergistically to inhibit the growth of Listeria monocytogenes Scott A.

### EXAMPLE II

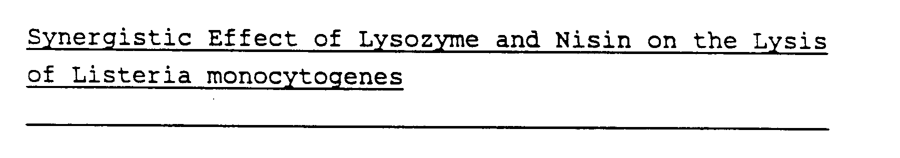

A culture of Listeria monocytogenes, Scott A, was grown overnight in BHI broth and harvested by centrifugation. The harvested cells were washed with 0.067M Potassium phosphate buffer (pH 6.6) and resuspended in 0.067M potassium phosphate buffer at various pH levels to a final absorbance of about 1.0 at 540 nm as determined in a Bausch & Lomb spectronic 20. Lysis of the bacteria is accompanied by a loss in absorbance at 540 nm. Nisin and lysozyme were added to these solutions to final concentrations of 100 IU/ml nisin and 10 µg/ml lysozyme and the solutions were placed in a 37° water bath whereupon the change in adsorption at 540 nm was monitored.

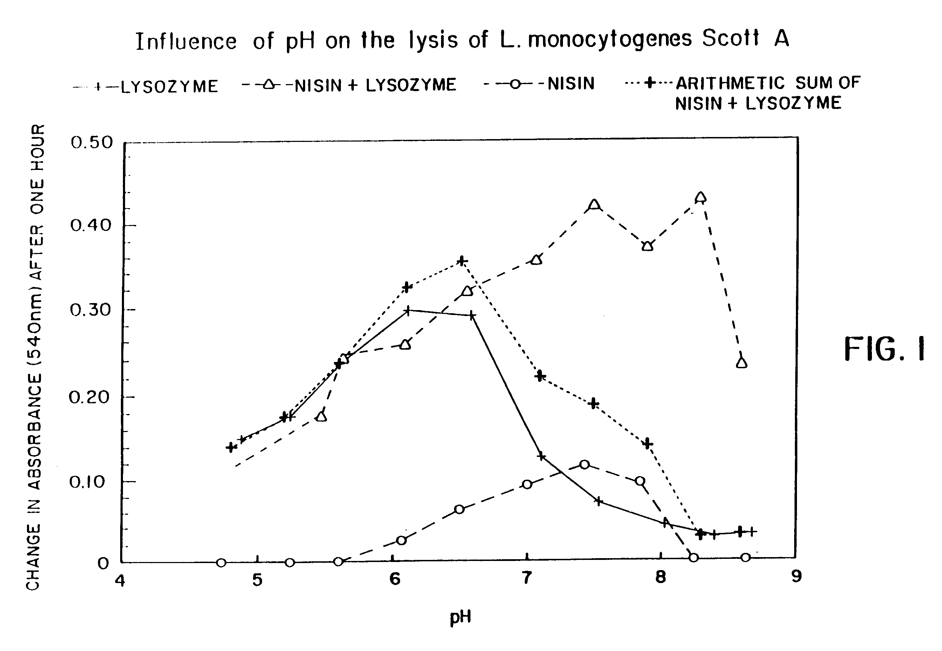

The results of this experiment are graphically set out in Figure 1. No significant change was evident in a nisin or lysozyme-free control after 5 hours. Lysis of L. monocytogenes by nisin alone was minimal below pH 5.5 or above pH 8.3, with maximum lysis occuring between pH 7 and 8. Lysis with lysozyme alone had its maximum at pH 6.0 - 6.5. At pH levels above 6.5, a definite synergistic effect between lysozyme and nisin was observed with these particular concentrations of the antimicrobials. The arithmetic sum of lysis by nisin and lysozyme is set out in the graph for comparison purposes. The comparison illustrates the synergistic effect produced by the nisin/lysozyme combination.

These results illustrate the synergism of nisin and lysozyme at various pH levels, particularly at the pH levels where the individual components are not considered to have antimicrobial activity in their own right. This is important even in systems where the pH is normally kept at low levels such as in cheese. Although the pH of cheese is usually low, the outer edges of surface ripened cheese often approach neutrality due to the action of ripening organisms in the cheese. When this happens, food-borne pathogens, such as L. monocytogenes, can grow on the surface of the cheese wheel. Incorporation of nisin/lysozyme at the levels previously mentioned into the cheese by surface application or their addition to the cheese milk controls this microbial growth.

### EXAMPLE III

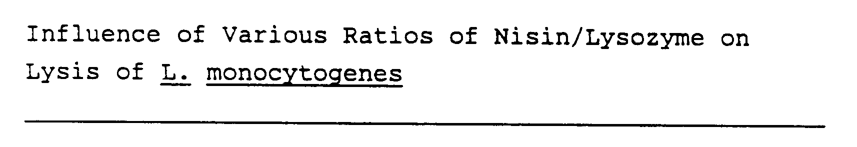

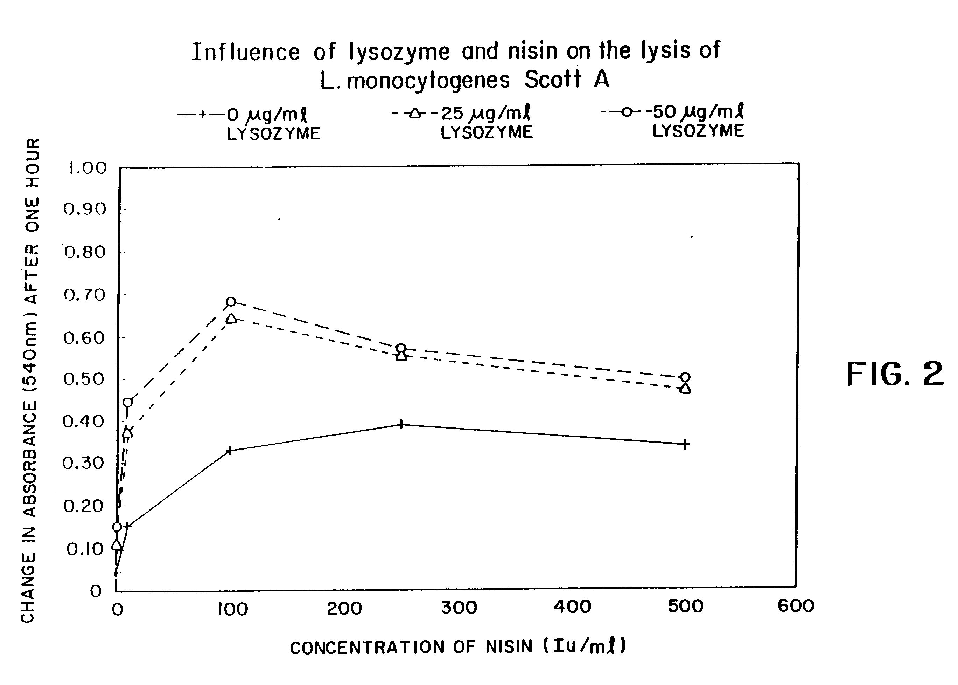

L. monocytogenes, Scott A strain, was grown as previously described, washed and resuspended in potassium phosphate buffer at pH 7.0. The change in absorbance after 1 hour of incubation at 37°C of various culture preparations is set out in Figure 2. From the data presented in this graphical represenation, it can be determined that it is possible to use significantly less nisin and lysozyme in combination than either of these materials by themselves to achieve a given amount of cell lysis.

For optimal lysis of the bacteria by nisin alone, a nisin concentration of about 200 IU/ml is called for. In the case of lysozyme, concentrations in excess of 50 µg/ml (usually about 100 µg/ml) are required. It can be determined from Figure 2 that the same rate of lysis achieved by 200 IU/ml of nisin can be achieved with about 10 IU of nisin in combination with about 25 µg/ml lysozyme. It can also be determined from Figure 2 that the extent of lysis achieved with less than 10 IU/ml nisin and 25 µg/ml lysozyme is greater than that achieved with 200 IU/ml nisin or 50 µg/ml lysozyme used individually. This represents a potentially significant cost savings because, historically, nisin has sold for about twice the price of lysozyme. Both products are relatively expensive by food-ingredients standards causing the use of nisin at 200 IU/g or lysozyme at 100 µg/ml to be prohibitively expensive in many applications. The present invention provides a means for achieving a significant reduction in the use of nisin and lysozyme resulting in a treatment cost of only 10% of the predicted cost of using nisin alone and less than 45% of the cost occurred when lysozyme is the sole anti-microbial agent.

### EXAMPLE IV

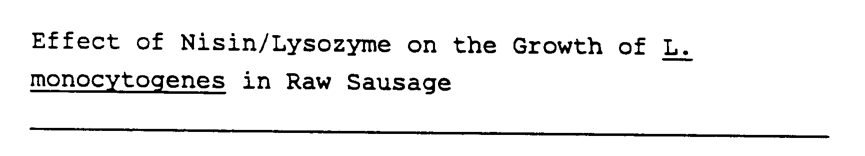

L. monocytogenes can grow at 5°C and poses a threat to some processed meats. Its control can include reduction in the rate of growth, bacteriostatic effects, and most desirably, bacteriocidal effects. The synergy of the nisin/lysozyme combination was demonstrated in a complex food system (raw pork sausage) using a high level of inoculation (over 10⁵ bacteria/gram) to illustrate the utility of the present invention under worst-case conditions.

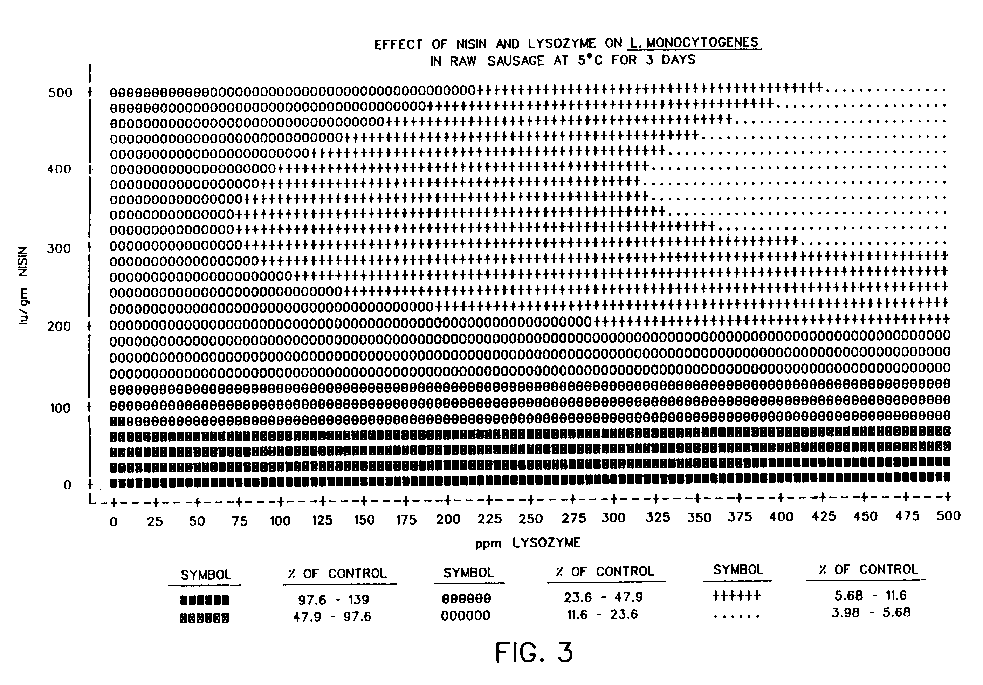

Raw sausage was inoculated with L. monocytogenes from a BHI overnight culture; various amounts of nisin and/or lysozyme were incorporated into the sausage. The treated sausage was held for either 3 or 21 days at 5°C whereupon viable L. monocytogenes were enumerated. A surface response model was constructed over a range of 0-500 IU/gm nisin and 0-500 ppm lysozyme. The surface response model from the 3 day data is set out in the following Figure 3. The figure demonstrates that lysozyme alone was ineffective for the control of L. monocytogenes in this food system, while nisin alone had some inhibitory effect. Significantly, the results show that some of the nisin can be replaced with lysozyme with the same level of control being maintained, and in some instances, significantly better performance was only achieved with the combination. For example, Figure 3 shows that no amount of nisin or lysozyme alone was able to reduce the Listeria level to 5.68 to 11.6% of the level of the untreated control in three days, but a mixture of about 300 IU/gram nisin and 75 ppm lysozyme was able to achieve this level of control. Similar synergies are seen at other levels of nisin and lysozyme. Thus, for any desired level of control, high concentrations of nisin can be replaced by significantly lower concentrations of nisin combined with lysozyme. These results were unexpected, especially since lysozyme alone was ineffective in this food system.

## Claims

1. A method of eliminating or inhibiting the growth of undesirable microorganisms in an environment amenable to their growth which comprises adding to such environment a synergistically effective combination of nisin and lysozyme in an amount sufficient to eliminate or inhibit such microbial growth.

2. The method of claim 1 wherein the lysozyme is a n-acetylhexosaminidase derived from the whites of chicken eggs.

3. The method of claim 1 wherein the lysozyme is an n-acetylhexosaminodase derived from human, bovine, T4 bacteriophage, or conventional or recombinant sources.

4. The method of claim 1 wherein the nisin is produced by a conventional or recombinant microorganism.

5. The method of claim 1 wherein the nisin is produced by pure culture fermentation of a nisin producing strain of Lactococcus lactis.

6. The method of claim 1 wherein the microorganism is selected from the group consisting of gram positive bacteria, gram negative bacteria and fungi.

7. The method of claim 5 wherein the nisin and/or lysozyme is provided by a nisin and/or a lysozyme producing organism growing in an environment in which the microbial growth is to be controlled.

8. The method of claim 1 wherein the concentration of nisin in the environment in which microbial growth is to be controlled is from 25 IU/ml to 2000 IU/ml and the ratio of nisin:lysozyme is from 9:1 to 1:2.

9. The method of claim 8 wherein the concentration of nisin is from 100 to 500 IU/ml.

10. The method of claim 1 wherein the nisin/lysozyme combination is added to a food product to control microbial growth therein.

11. The method of claim 1 wherein the undesirable microorganism is a bacterium of the species Listeria monocytogenes.

12. The method of claim 11 wherein the L. monocytogenes is a Scott A. strain.

13. A method of controlling the growth of L. monocytogenes in foodstuffs which method involves combining such foodstuffs with a combination of nisin/lysozyme wherein the nisin is provided in an amount of from 100 to 500 IU/ml (or the weight equivalent thereof) and the ratio of nisin:lysozyme is from 9 IU/ml:1 µg/ml to 1 IU/ml:2 µg/ml.

14. The method of claim 13 wherein the ratio of nisin to lysozyme is 2:1.

## Patentansprüche

1. Verfahren zur Ausschaltung oder Hemmung des Wachstums unerwünschter Mikroorganismen in einer für ihr Wachstum geeigneten Umgebung, umfassend das Hinzufügen von einer synergistisch wirksamen Kombination aus Nisin und Lysozym zu solch einer Umgebung in einer Menge, die ausreicht, um solches mikrobielles Wachstum auszuschalten- oder zu hemmen.

2. Verfahren nach Anspruch 1, worin das Lysozym eine N-Acetylhexosaminodase ist, die aus Hühnereiweiß stammt.

3. Verfahren nach Anspruch 1, worin das Lysozym eine N-Acetylhexosaminodase ist, die vom Menschen, Rind, T4-Bakteriophage oder herkömmlichen oder rekombinanten Quellen stammt.

4. Verfahren nach Anspruch 1, worin das Nisin durch einen herkömmlichen oder rekombinanten Mikroorganismus produziert wird.

5. Verfahren nach Anspruch 1, worin das Nisin durch reine Kulturfermentation eines Nisin erzeugenden Stammes von Lactococcus lactis produziert wird.

6. Verfahren nach Anspruch 1, worin der Mikroorganismus aus der Gruppe, die aus grampositiven Bakterien, gramnegativen Bakterien und Pilzen besteht, ausgewählt ist.

7. Verfahren nach Anspruch 5, worin das Nisin und/oder Lysozym von einem Nisin und/oder Lysozym erzeugenden Organismus bereitgestellt wird, der in einer Umgebung wächst, in der das mikrobielle Wachstum kontrolliert werden soll.

8. Verfahren nach Anspruch 1, worin die Nisinkonzentration in der Umgebung, in der mikrobielles Wachstum kontrolliert werden soll, von 25 IU/ml bis 2000 IU/ml reicht und das Verhältnis von Nisin:Lysozym von 9:1 bis 1:2 reicht.

9. Verfahren nach Anspruch 8, worin die Nisinkonzentration von 100 bis 500 IU/ml reicht.

10. Verfahren nach Anspruch 1, worin die Nisin/Lysozym Kombination zu einem Nahrungsmittelprodukt hinzugegeben ist, um das mikrobielle Wachstum darin zu kontrollieren.

11. Verfahren nach Anspruch 1, worin der unerwünschte Mikroorganismus ein Bakterium der Spezies Listeria monocytogenes ist.

12. Verfahren nach Anspruch 11, worin L. monocytogenes ein Scott A Stamm ist.

13. Verfahren zum Kontrollieren des Wachstums von L. monocytogenes in Nahrungsmitteln, wobei das Verfahren das Kombinieren solcher Nahrungsmittel mit einer Kombination aus Nisin/Lysozym umfaßt, worin das Nisin in einer Menge von 100 bis 500 IU/ml (oder dem Gewichtsäquivalent davon) zur Verfügung gestellt ist und das Verhältnis von Nisin:Lysozym von 9 IU/ml:1 µg/ml bis 1 IU/ml:2 µg/ml reicht.

14. Verfahren nach Anspruch 13, worin das Verhältnis Nisin zu Lysozym 2:1 ist.

## Revendications

1. Méthode d'élimination ou d'inhibition de la croissance de microorganismes indésirables dans un environnement susceptible d'une telle croissance qui comprend l'addition à un tel environnement d'une combinaison synergiquement efficace de nisine et de lysozyme en quantité suffisante pour éliminer ou inhiber une telle croissance microbienne.

2. Méthode selon la revendication 1 dans laquelle le lysozyme est une n-acétylhexosaminidase dérivée de blancs d'oeufs de poule.

3. Méthode selon la revendication 1 dans laquelle le lysozyme est une n-acétylhexosaminidase dérivée de bactériophages T4, humains ou bovins ou de sources classiques ou recombinantes.

4. Méthode selon la revendication 1 dans laquelle la nisine est produite par un microorganisme classique ou recombinant.

5. Méthode selon la revendication 1 dans laquelle la nisine est produite par une fermentation en culture pure d'une souche de Lactococcus lactis productrice de nisine.

6. Méthode selon la revendication 1 dans laquelle le microorganisme est choisi dans le groupe composé de bactéries gram-positives, de bactéries gram-négatives et de champignons.

7. Méthode selon la revendication 5 dans laquelle la nisine et/ou le lysozyme est fourni par un organisme producteur de nisine et/ou de lysozyme se développant dans un environnement dans lequel la croissance microbienne doit être contrôlée.

8. Méthode selon la revendication 1 dans laquelle la concentration de nisine dans l'environnement dans lequel la croissance microbienne doit être contrôlée va de 25 IU/ml à 2000 IU/ml et le taux nisine:lysozyme va de 9:1 à 1:2.

9. Méthode selon la revendication 8 dans laquelle la concentration de nisine est de 100 à 500 IU/ml.

10. Méthode selon la revendication 1 dans laquelle la combinaison de nisine/lysozyme est ajoutée à un produit alimentaire pour contrôler la croissance microbienne dans ce dernier.

11. Méthode de la revendication 1 dans laquelle le microorganisme indésirable est une bactérie de l'espèce Listeria monocytogenes.

12. Méthode selon la revendication 11 dans laquelle la L. monocytogenes est une souche Scott A.

13. Méthode de contrôle de la croissance de la L. monocytogenes dans des denrées alimentaires qui implique la combinaison de telles denrées alimentaires avec une combinaison de nisine/lysozyme dans laquelle la nisine est amenée dans une quantité de 100 à 500 IU/ml (ou l'équivalent en poids) et le taux de nisine:lysozyme est de 9 IU/ml:1 µg/ml à 1 IU/ml:2 µg/ml.

14. Méthode selon la revendication 13 dans laquelle le taux de nisine:lysozyme est de l'ordre de 2:1.
